# EUROPEAN PATENT APPLICATION

(11) **EP 1 106 060 A1**
(43) Date of publication of application: **13.06.2001**
(21) Application number: 99938511.5
(22) Date of filing: 18.08.1999
(51) Int. Cl.: A01K 67/027, G01N 33/50, G01N 33/15, A61K 45/00

(54) **MODEL ANIMAL WITH VISUALIZED NERVE NETWORK**

(30) Priority: 19.08.1998 JP 23281798
(71) Applicant: CHUGAI SEIYAKU KABUSHIKI KAISHA, Tokyo, 115-8543 (JP)
(72) Inventor: YOSHIHARA, Yoshihiro, Osaka-shi, Osaka 533-0012 (JP)
(74) Representative: Maschio, Antonio
(86) International application number: JP9904439
(87) International publication number: WO0010382

(57) **Abstract**

The present invention provides a transgenic animal into which a gene encoding a trans-synaptic tracer protein is introduced so as to direct specific expression in particular neurons. The use of this transgenic animal permits the selective visualization of functional neural pathways through a particular group of neurons, which could not have been achieved by tracing technique using a conventional trans-synaptic tracer protein.

## Description

### Filed of the Invention

The present invention relates to transgenic animals into which a gene encoding a trans-synaptic tracer protein is introduced so as to direct specific expression in particular types of neurons, a method for screening neuromimetic substances using the transgenic animals, and a neuromimetic substance obtainable by the screening method.

### Prior Art

The brain with its various functions including learning, memory, multisensory recognition and integration, motor development and control, as well as emotion is composed of complex, but well-ordered neural networks. To study brain structure and function, there is a need to understand the molecular mechanisms for formation, maintenance and plasticity of neural pathways. In particular, it is undoubtedly the touchstone of studies in these various areas of neuroscience to elucidate how neurons extend their axons in the correct direction, how they recognize target cells, how they form synapses, how they form and maintain neural networks, and how they further plastically change the formed neural pathways as needed.

A variety of plant lectins have been conventionally used as trans-synaptic tracers in neuroanatomical studies on neuronal connectivity. In particular, wheat germ agglutinin (WGA) has been most efficiently transferred from primary neurons to secondary neurons across synapses, thereby exhibiting its usefulness in any neural systems. In the visual system, for example, WGA injected into one eye is taken up by ganglion cells of the retina and then transported through optic nerves to the lateral geniculate nucleus of the thalamus, where WGA is trans-synaptically transferred to thalamic secondary neurons, resulting in a WGA-labeled visual cortical area which is the projection site of the thalamic secondary neurons. In this way, the ocular dominance columns can be visualized. Thus, the technique using WGA as a tracer is very useful and powerful, and has greatly contributed to the development of neuroscience.

However, the above conventional tracing technique using WGA does not allow selective visualization of functional neural pathways through a particular group of neurons because WGA was taken up by all the cells surrounding the site of WGA injection. In addition, other problems have also been pointed out, for example, serious immune responses induced in a WGA-injected animal due to the recognition of WGA as a foreign substance.

### Problems to be solved by the Invention

The tracing technique using WGA is very useful for studying functional connectivity patterns between neurons, but it also involves the various problems mentioned above. The object of the present invention is to overcome these problems.

### Means for solving the Problems

Our research efforts were directed to overcoming the above problems, and we have found that these problems can be overcome by using a transgenic animal into which a gene encoding a trans-synaptic tracer protein is introduced so as to direct specific expression in particular neurons, thereby finally completing the invention.

Thus, the present invention provides transgenic animals into which a gene encoding a trans-synaptic tracer protein is introduced so as to direct specific expression in particular neurons.

The present invention also provides a method for screening neuromimetic substances, which comprises administering a test substance to the transgenic animal mentioned above, and selecting a neuromimetic substance from among the test substances by using as an indicator the trans-synaptic tracer protein expressed in the animal's neurons.

The present invention further provides a neuromimetic substance obtainable by the screening method mentioned above.

This specification includes part or all of the contents as disclosed in the specification and/or drawings of Japanese Patent Application No. 10-232817 which is a priority document of the present application.

### Disclosure of the Invention

The present invention will now be described in more detail.

The transgenic animal of the present invention is characterized in that a gene encoding a trans-synaptic tracer protein is introduced so as to direct specific expression in particular neurons.

In addition to WGA mentioned above, examples of the trans-synaptic tracer protein include, but are not limited to, Concanavalin A agglutinin (ConA), Pisum Sativum agglutinin (PSA), Lens Culinaris agglutinin (LCA) and the like. To express a gene encoding a trans-synaptic tracer protein in particular neurons, a promoter specific to the particular neurons may be connected upstream of the gene, but any other technique can be used for this purpose. The promoter specific to particular neurons includes, but is not limited to, cerebellar Purkinje cell-specific L7 promoter, olfactory receptor cell-specific OMP promoter and the like. As used in the transgenic animals of the present invention, the term "specific" or "specifically" means that the trans-synaptic tracer protein is sufficiently expressed to distinguish particular neurons from other cells when it is visualized with an enzyme-labeled antibody etc., but it does not necessarily mean that no trans-synaptic tracer protein gene is expressed in any other cell.

A wild-type gene encoding the trans-synaptic tracer protein may be used without any further modification. For the WGA gene, the modified gene that lacks a C-terminal propeptide-coding segment may be preferably used for the reason described below.

Any animal having neural pathways may be used in the present invention.

The transgenic animal of the present invention may be constructed as follows. A fragment containing the promoter specific to particular neurons and a fragment containing the trans-synaptic tracer protein gene may be amplified by PCR, respectively, and these amplified fragments may then be inserted into an existing vector for recombination. The resulting recombinant vector may be injected into fertilized eggs or embryos from recipient animals. A transgenic animal having the trans-synaptic tracer protein gene may be selected out of the resulting animals. For the L7 promoter, the fragment containing the promoter specific to particular neurons may be obtained by preparing primers that can amplify a region upstream from the initiation codon of the nucleotide sequence shown in SEQ ID NO: 2; amplifying a part of the promoter region by PCR using mouse genomic DNA as a template; and then screening a mouse genomic DNA library (e.g., commercially available mouse Genomic DNA Library SC945301 (Stratagene)) using the amplified PCR product as a probe. For the OMP promoter, such a fragment may be obtained by preparing a primer that can amplify a region upstream from the initiation codon of the nucleotide sequence shown in SEQ ID NO: 3; and carrying out PCR using mouse genomic DNA as a template. For the WGA gene, the fragment containing the trans-synaptic tracer protein gene may be obtained by preparing a primer that can amplify a coding-region of the nucleotide sequence shown in SEQ ID NO: 1; and carrying out PCR using wheat germ cDNA as a template.

The transgenic animal of the present invention is useful for the elucidation of causes for various neurogenic diseases and the establishment of medical treatment for these diseases. For example, the transgenic animal of the present invention may be crossed with an animal model for diseases resulting from abnormal neural pathways or with a spontaneously mutated animal model. The resulting animal may then be used to analyze the abnormal neural pathways responsible for the diseases or compensatory pathways induced by the diseases. The transgenic animal of the present invention may also be used to create an artificial pathological model for Parkinson's disease, ischaemia, head injury or various mental diseases for the analysis of injured pathways or compensatory pathways. Further, the transgenic animal of the present invention developing pathological conditions may be administered with various drugs in order to assess the potency of the administered drugs, i.e., their ability to restore injured pathways or to form compensatory pathways, by using trans-synaptic tracer protein as an indicator.

In addition, the transgenic animal's tissues expressing the trans-synaptic tracer protein may be primarily cultured to create cultured neurons expressing the trans-synaptic tracer protein, which may then be used for the screening of drugs that affect cell survival and maintenance, dendrite extension, synapse formation, various enzymatic activities, and/or neurotransmitter production.

The neuromimetic substance of the present invention may be obtained according to the above screening methods, for example, by screening test substances such as peptides, proteins, non-peptide compounds, synthetic compounds, fermented products from microorganisms, marine organism extracts, plant extracts, cell extracts, or animal tissue extracts. These test substances may be novel or known compounds.

The present invention will be further described in the following example. The example is provided for illustrative purposes only, and is not intended to limit the scope of the invention.

### Examples

### Example 1: Construction of a vector expressing WGA

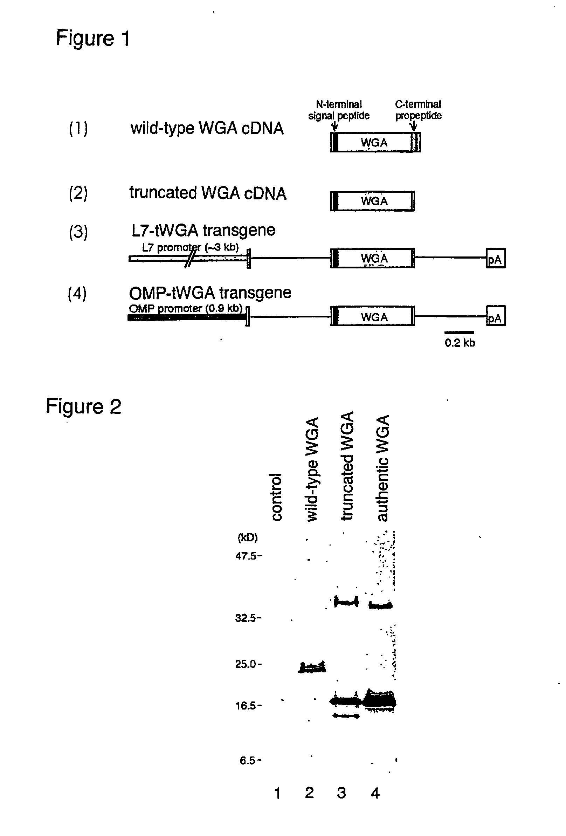

A wild-type WGA cDNA insert (1.0 kb) excised from pWGA-D (Smith, J. J. & Raikhel, N.V., Plant Mol. Biol. 13, 601-603 (1989)) was blunt-ended, followed by addition of a BstX I site. This insert was subcloned into a BstX I site of a mammalian expression vector pEF-BOS (Mizushima, S. & Nagata, S., Nucl. Acids Res. 18, 5322 (1990)) to construct a plasmid pEF-WGA. Mouse nueroblastoma N2a cells were transfected with this plasmid using Lipofectamine and Opti-MEM (Gibco/BRL). After 48 hours of transfection, the cells were tested for the presence of expressed WGA by Western blotting using anti-WGA antibody. As shown in Figure 2, WGA was detected (lane 2, MW 24kD), but it had a significantly larger size than that of an authentic WGA (lane 4, MW 18 kD).

Since the plant WGA has a C-terminal propeptide (15 amino acid residues) which is involved in selective delivery of the lectin into vacuoles (Broadwell, R. D. & Balin, B. J., J. Comp. Neurol. 242, 632-650 (1985)), the difference in molecular weight was thought to be caused by the absence of C-terminal propeptide-processing mechanism in animal cells. Thus, we decided to construct a plasmid containing a DNA segment encoding a truncated WGA which lacks the C-terminal propeptide.

A plasmid pEF-tWGA containing the truncated WGA-coding cDNA (Figure 1(2)) was constructed from the plasmid pEF-WGA by replacing a codon GTC (valine 198) in the wild-type WGA-coding DNA (Figure 1(1)) with an opal stop codon TGA by PCR mutagenesis using not-completely complementary primers. The N2a cells were transfected with this plasmid pEF-tWGA and then tested for the presence of expressed WGA by Western blotting, thereby detecting the truncated WGA having the same size as the authentic WGA (Figure 2, lane 3). In addition, the amount of the truncated WGA produced was significantly larger than that of the wild-type WGA. Thus, we decided to use this truncated WGA-coding DNA in all the following experiments.

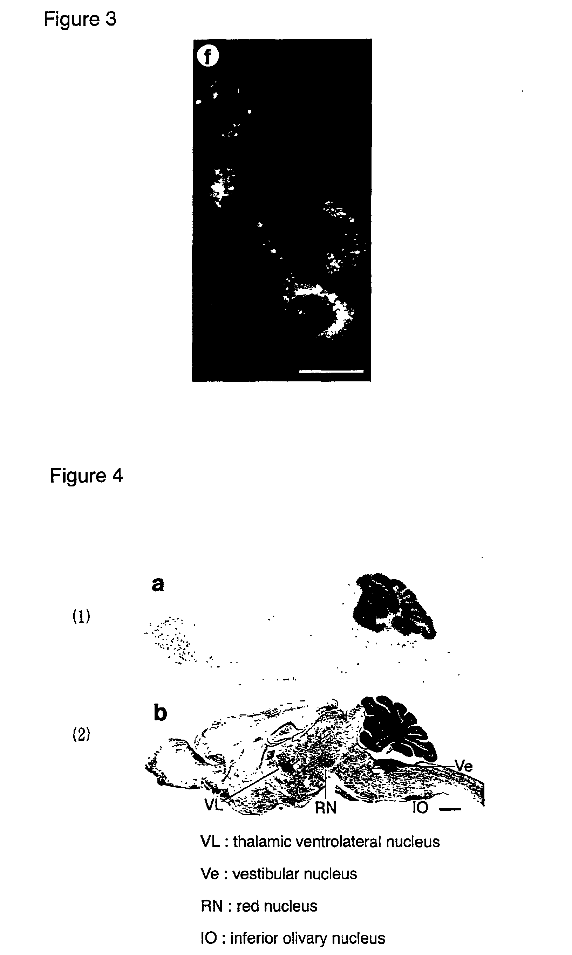

The N2a cells transfected with pEF-tWGA were treated with anti-WGA polyclonal antibody and Cy3 anti-rabbit antibody IgG (Jackson) (10 µg/ml, Sigma), followed by observation using a confocal laser scanning microscopy system (Bio-Rad MRC-600) equipped with Zeiss Axiophot F1 microscope (Figure 3). As shown in Figure 3, the truncated WGA strongly bound to the intracellular granule-like structures of N2a cells.

### Example 2: Construction of a pL7-tWGA-introduced mouse

A mouse L7 promoter region (3.5 kb) was amplified from Pcp2-z06 plasmid (Vandaele, S. et al., Genus Dev. 5, 1136-1148 (1991)). L7 (Pcp2) gene promoter has been analyzed in detail (Oberdick, J. et al., Neuron 1, 367-376 (1988); Oberdick, J. et al., Science 248, 223-226 (1990); Oberdick, J. et al., Neuron 10, 1007-1018 (1993); Vandaele, S. et al., Genus Dev. 5, 1136-1148 (1991)), and used for cerebellar Purkinje cell-specific expression of foreign genes (Feddersen, R. M. et al., Neuron 9, 955-966 (1992); Burright, E. N. et al., Cell 82, 937-948 (1995)). This amplified fragment was subcloned into a blunt-ended BamH I site of pBstN vector, which contains human β-globin gene introns and SV 40 polyadenylation signal. A tWGA cDNA sequence (0.6 kb) excised from pEF-tWGA was ligated to a blunt-ended EcoR I site of the pBstN vector to construct a plasmid pL7-tWGA for cerebellar Purkinje cell-specific expression of the truncated WGA (Figure 1(3)).

The purified pL7-tWGA was injected into the male pronucleus of fertilized eggs from FVB/N mice (CLEA Japan), mainly according to the procedures described by Nohmi, T. et al., Environment. Mol. Mutagenesis 28, 465-470 (1996). The pL7-tWGA-injected eggs were cultured and transferred into the oviduct of ICR pseudopregnant recipients (CLEA Japan). Tail samples taken for DNA analysis were screened for the integrated transgene by PCR and Southern analysis, thereby obtaining a transgenic mouse having the full-length transgene.

The presence of expressed WGA mRNA and protein in this transgenic animal's brain was determined by in situ hybridization and immunohistochemistry, respectively.

In situ hybridization was carried out according to the procedures described by Yoshihara, Y. et al., J. Neurosci. 17, 5830-5842 (1997) as follows.

Sections (50 µm) of an adult mouse brain perfused with paraformaldehyde were treated with proteinase K (10 µg/ml at 25 °C for 30 min), acetylated, dehydrated, and then air-dried. An antisense riboprobe for WGA (540 nucleotides in length) was prepared using ³⁵S-UTP (Amersham) and an RNA transcription kit (Stratagene). The sections were hybridized overnight with the above antisense riboprobe (1 × 10⁶ cpm/ml) in a humidified chamber at 56 °C. After hybridization, the sections were washed with 4 × SSC, treated with RNase A (10 µg/ml at 37 °C for 30 min), washed with 0.05 × SSC, dehydrated with ethanol, and then exposed to βmax X-ray film (Amersham).

Also, immunohistochemistry was carried out as follows.

Sections (50 pm) of a mouse brain perfused with paraformaldehyde were cut with a sliding microtome, pre-treated with 0.3% H₂O₂, blocked, and then incubated for 2 to 24 hours at room temperature with anti-WGA polyclonal antibody (3 µg/ml, Sigma) which had been absorbed with 1% acetone powder of mouse brain. The sections were then incubated either with biotin anti-rabbit IgG (Zymed), followed by a Vectastain ABC elite kit (Vector), or with horseradish peroxidase anti-rabbit IgG (Jackson). The generated signals were visualized through the Ni²⁺-enhanced diaminobenzidine/peroxide reaction for analysis using a transmission microscopy system.

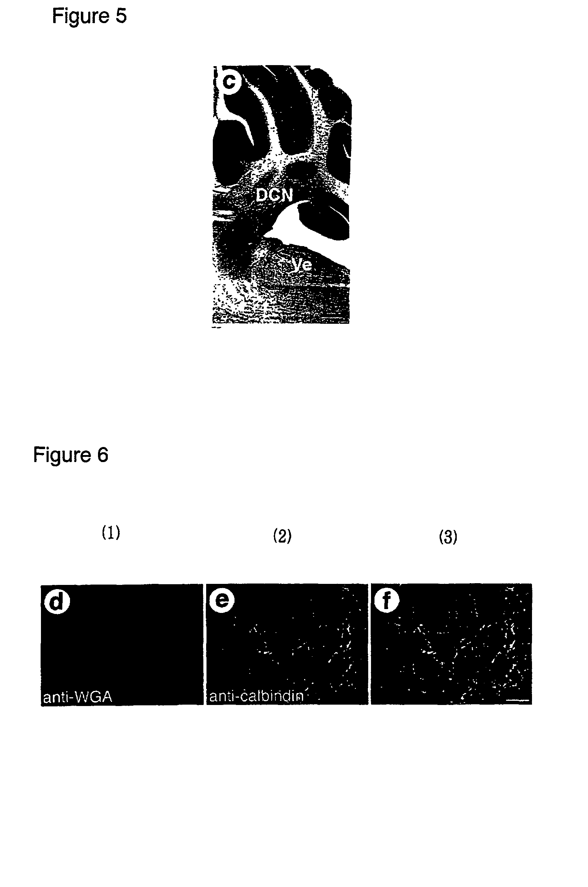

Figure 4 (1) shows WGA mRNA detection in a section including the whole brain tissue. Figure 4 (2) shows WGA protein detection in an adjacent section to the section shown in Figure 4 (1). As shown in both figures, WGA mRNA detection is limited to the Purkinje cells, while the WGA protein is expressed not only in the Purkinje cells, but also in other cells anatomically and functionally associated with the Purkinje cells. Figure 5 shows WGA protein detection in a section including the cerebellum, indicating that the WGA protein is expressed in the deep cerebellar nuclei (dentate, fastigial, interposed) and the vestibular nucleus, as well as the Purkinje cells.

Axons of the Purkinje cells form synapses with neurons in the deep cerebellar nuclei (Ito, M., The cerebellum and neural control., New York, Raven Press (1984); Altman, J. & Bayer, S.A., Development of the cerebellar system: in relation to its evolution, structure, and functions., Boca Raton, Florida, CRC Press (1996)). Since some Purkinje cells directly project to the vestibular nucleus, secondary neurons are also present in the vestibular nucleus. In view of the foregoing, WGA is thought to be transported to secondary and tertiary neurons of the Purkinje cells.

Double immunofluorescence labeling was used to determine the presence of Purkinje cells and expressed WGA protein in the cerebellum of the transgenic mouse transformed with pL7-tWGA. The Purkinje cells were detected by anti-calbindin antibody (Sigma) and FITC anti-mouse IgG (Cappel). Calbindin is specifically found in the Purkinje cells. The WGA protein was detected by anti-WGA antibody and Cy3 anti-rabbit IgG (Jackson). Figures 6 (1) and (2) show WGA protein and Calbindin in the deep cerebellar nuclei, respectively. Figure 6 (3) shows the detection of both. These figures indicate the trans-synaptic transfer of WGA protein from axon termini of the Purkinje cells to neurons in the deep cerebellar nuclei.

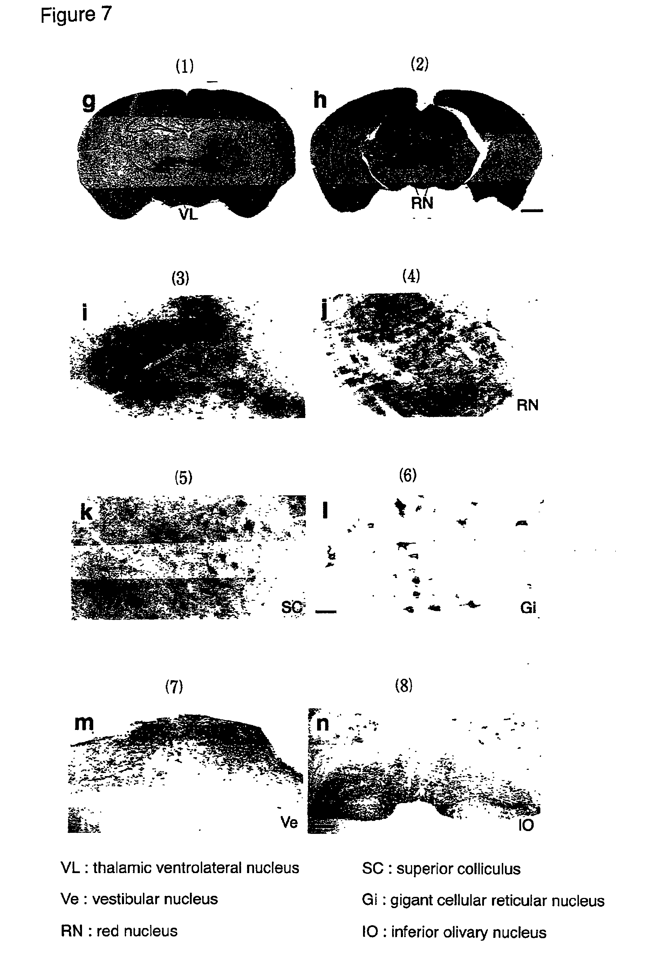

Immunohistochemistry with anti-WGA antibody was used to determine the presence of expressed WGA protein in brain parts other than the cerebellum of the transgenic mouse transformed with the plasmid pL7-tWGA. Figures 7 (1) and (2) show WGA protein detection in a section including the thalamic ventrolateral nucleus and in a section including the red nucleus, respectively. Figures 7 (3) and (4) show magnified views of Figures 7 (1) and (2), respectively. Figures 7 (5) to (8) show WGA protein detection in a section including the superior colliculus, in a section including the gigant cellular reticular nucleus, in a section including the vestibular nucleus, and in a section including the inferior olivary nucleus, respectively. These figures indicate that the WGA protein is detected in any of the thalamic ventrolateral nucleus, red nucleus, superior colliculus, gigant cellular reticular nucleus, vestibular nucleus and inferior olivary nucleus. All of these neurons form synapses with axons from the deep cerebellar nuclei, and correspond to tertiary neurons of the Purkinje cells.

### Example 3: Construction of a pOMP-tWGA-introduced mouse

An OMP promoter region (0.9 kb; Buiakova, O. I. et al., Genomics 20, 452-462 (1994)) was amplified by PCR from mouse genomic DNA and subcloned into a blunt-ended BamH I site of pBstN vector (Figure 1 (4)). A tWGA cDNA sequence was inserted downstream of the OMP promoter region to obtain a plasmid pOMP-tWGA. This plasmid pOMP-tWGA was used to construct a transgenic mouse, as described in Example 2.

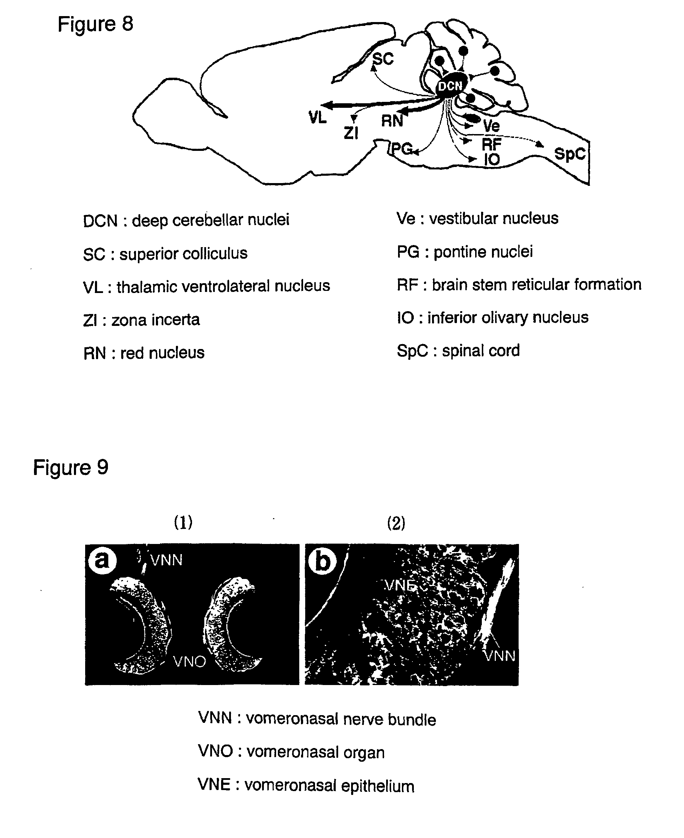

The presence of expressed WGA protein in the vomeronasal organ of the above transgenic mouse was determined by an immunofluorescence labeling technique using anti-WGA antibody and Cy3 anti-rabbit IgG. Figures 9 (1) and (2) show WGA protein detection in a section including the vomeronasal organ and a magnified view thereof, respectively, indicating that the WGA protein is highly expressed in the vomeronasal epithelium and nerve bundles thereof.

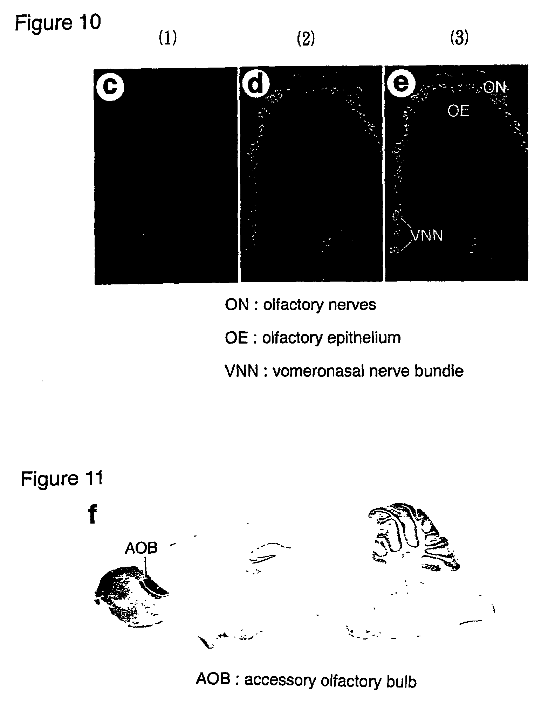

Double immunofluorescence labeling was used to determine the presence of axons and expressed WGA protein in the vomeronasal organ of the transgenic mouse transformed with pOMP-tWGA. The axons were detected by anti-NCAM antibody and FITC anti-mouse IgG (Cappel). NCAM is specifically found in the axons. The WGA protein was detected by anti-WGA antibody and Cy3 anti-rabbit IgG (Jackson). Figures 10 (1) and (2) show WGA protein detection and axon detection, respectively. Figure 10 (3) shows the detection of both. These figures indicate that NCAM is evenly expressed in the olfactory and vomeronasal nerves, while the WGA protein is highly expressed in the vomeronasal nerves, in particular.

Immunohistochemistry was used to determine the presence of expressed WGA protein in the brain of the transgenic mouse transformed with pOMP-tWGA.

Figure 11 shows WGA protein detection in a section including the whole brain, indicating that the WGA protein is highly expressed in the accessory olfactory bulb.

Figures 12 (1) and (2) show WGA protein detection in a section including the accessory olfactory bulb, indicating that the WGA protein is expressed not only in vomeronasal axon termini of the glomerulus, but also in external plexiform layer and granule cell layer thereof.

Figures 13 (1), (2) and (3) show WGA protein detection in a section including the lateral olfactory tract, indicating that the WGA protein is also expressed in mitral/ tufted cell axons of the lateral olfactory tract, which corresponds to axons of secondary neurons.

Figures 14 (1) and (4) show WGA protein detection in a section including the medial amygdaloid nucleus. Figures 14 (2) and (3) show WGA protein detection in a section including the posteromedial cortical amygdaloid nucleus and in a section including the bed nucleus of stria terminalis, respectively. These figures indicate that the WGA protein is expressed in any of the medial amygdaloid nucleus, posteromedial cortical amygdaloid nucleus, and bed nucleus of stria terminalis, which correspond to tertiary neurons of the vomeronasal cells.

All publications, patents and patent applications cited herein are incorporated herein by reference in their entirely.

### Effects of the Invention

The present invention permits the selective visualization of functional neural pathways through a particular group of neurons, which could not have been achieved by tracing technique using a conventional trans-synaptic tracer protein. Further, the present invention does not have any of the problems observed in the tracing technique using a conventional trans-synaptic tracer protein, for example, serious immune responses caused by injection of the trans-synaptic tracer protein into an animal and individual differences due to injection technique.

### Brief Description of the Drawings

Figure 1 shows the construction of WGA gene.

Figure 2 shows Western blotting of the WGA gene product (electrophoresis photograph).

Figure 3 shows WGA protein detection in N2a cells (microphotograph).

Figure 4 shows WGA mRNA detection and WGA protein detection in a section of the brain (microphotographs).

Figure 5 shows WGA protein detection in a section including the cerebellum (microphotograph).

Figure 6 shows WGA protein detection in the deep cerebellar nuclei (microphotographs).

Figure 7 shows WGA protein detection in various brain sections (microphotographs).

Figure 8 shows a schematic diagram of neural pathways originating from Purkinje cells.

Figure 9 shows WGA protein detection in a section including the vomeronasal organ (microphotographs).

Figure 10 shows axon detection and WGA protein detection in a section including axons of the vomeronasal organ (microphotographs).

Figure 11 shows WGA protein detection in a section of the brain (microphotograph).

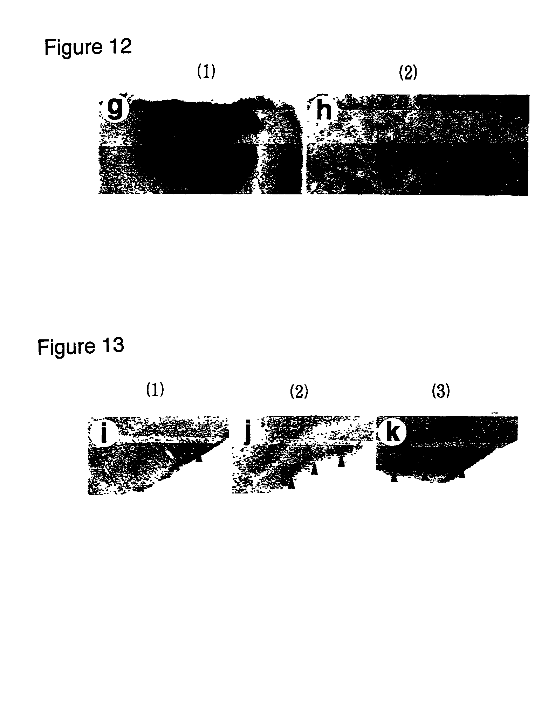

Figure 12 shows WGA protein detection in a section including the accessory olfactory bulb (microphotographs).

Figure 13 shows WGA protein detection in a section including the lateral olfactory tract (microphotographs).

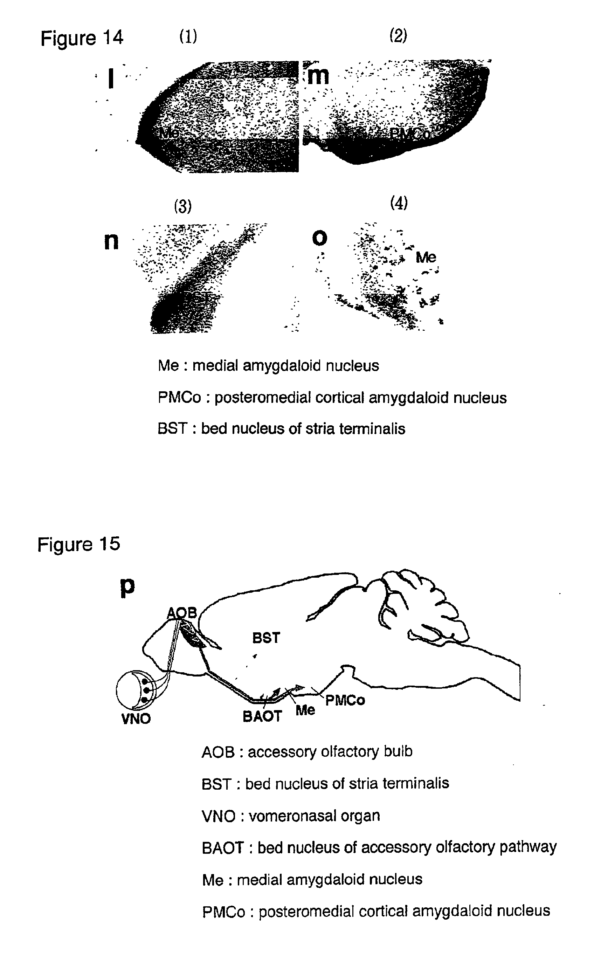

Figure 14 shows WGA protein detection in a section including the medial amygdaloid nucleus, posteromedial cortical amygdaloid nucleus, and bed nucleus of stria terminalis (microphotographs).

Figure 15 shows a schematic diagram of neural pathways originating from vomeronasal sensory neurons.

SEQUENCE LISTING
<110> CHUGAI SEIYAKU KABUSHIKI KAISYA

<120> AN ANIMAL MODEL WITH VISIBLE NEURAL NETWORKS

<130> PH-670-PCT

<150> JP 98/232817
<151> 1998-8-19

<160> 3

<170> PatentIn version 2.0

<210> 1
<211> 998
<212> DNA
<213> Triticum aestivum
accagcacca agaaaacaaa aagc atg aag atg atg agc acc agg gcc ctc 51
Met Lys Met Met Ser Thr Arg Ala Leu
1               5
gcg ctc ggc gcg gct gcc gtc ctc gcc ttc gcc gcg gcg acc gct cag 99
Ala Leu Gly Ala Ala Ala Val Leu Ala Phe Ala Ala Ala Thr Ala Gln
10                  15                  20                  25
gcc cag agg tgc ggc gag caa ggc agc aac atg gag tgc ccc aac aac 147
Ala Gln Arg Cys Gly Glu Gln Gly Ser Asn Met Glu Cys Pro Asn Asn
                30                  35                  40
ctc tgc tgc agc cag tac ggc tac tgc ggg atg ggc ggc gac tac tgc 195

Leu Cys Cys Ser Gln Tyr Gly Tyr Cys Gly Met Gly Gly Asp Tyr Cys
            45                  50                  55
ggc aag ggc tgc cag aac ggc gcc tgc tgg acc agc aag cgc tgc ggc 243
Gly Lys Gly Cys Gln Asn Gly Ala Cys Trp Thr Ser Lys Arg Cys Gly
        60                  65                  70
agc cag gcc ggc ggc gcg acg tgc acc aac aac cag tgc tgc agc cag 291
Ser Gln Ala Gly Gly Ala Thr Cys Thr Asn Asn Gln Cys Cys Ser Gln
    75                  80                  85
tac ggg tac tgc ggc ttc ggc gcc gag tac tgc ggc gcc ggc tgc cag 339
Tyr Gly Tyr Cys Gly Phe Gly Ala Glu Tyr Cys Gly Ala Gly Cys Gln
90                  95                  100                 105
ggc ggc ccc tgc cgc gcc gac atc aag tgc ggc agc cag gcc ggc ggc 387
Gly Gly Pro Cys Arg Ala Asp Ile Lys Cys Gly Ser Gln Ala Gly Gly
                110                 115                 120
aag ctg tgc ccg aac aac ctc tgc tgc agc cag tgg gga ttc tgc ggc 435
Lys Leu Cys Pro Asn Asn Leu Cys Cys Ser Gln Trp Gly Phe Cys Gly
            125                 130                 135
ctc ggt tcc gag ttc tgc ggc ggc ggc tgc cag agc ggt gct tgc agc 483
Leu Gly Ser Glu Phe Cys Gly Gly Gly Cys Gln Ser Gly Ala Cys Ser
        140                 145                 150
acc gac aaa ccg tgc ggc aag gac gcc ggc ggc aga gtt tgc act aac 531
Thr Asp Lys Pro Cys Gly Lys Asp Ala Gly Gly Arg Val Cys Thr Asn
    155                 160                 165
aac tac tgt tgt agc aag tgg gga tcc tgt ggc atc ggc ccg ggc tat 579
Asn Tyr Cys Cys Ser Lys Trp Gly Ser Cys Gly Ile Gly Pro Gly Tyr
170                 175                 180                 185
tgc ggt gca ggc tgc cag agt ggc ggc tgc gat ggt gtc ttc gcc gag 627
Cys Gly Ala Gly Cys Gln Ser Gly Gly Cys Asp Gly Val Phe Ala Glu
                190                 195                 200

gcc atc acc gcc aac tcc act ctt ctc caa gaa tgatgatcaa tcttgctatg 680
Ala Ile Thr Ala Asn Ser Thr Leu Leu Gln Glu
            205                 210
gcagtattgc aacgacgaat aatccgtggc aatctcattg ccacctacgg tttcccttga 740
cttactttta gagtactagt ccttaataat tctctagctt gcaatatgat gtgcaggtta 800
ctgcagcaga aacaaaatat tgctgtcgtg catgcatgga aatattgcag tgagaaagta 860
ctgtgtggca atatagggtg tgctattgtt gccgcaaatt agttttcttg ttatgacctg 920
ttgtcaggat gcatgcatgg ctgttgtaat gttggagtac ttcgtgattt cgttgcaata 980
tattaccatg gttctcac 998

<210> 2
<211> 3935
<212> DNA
<213> Mus musculus

<400> 2
gcttaactgg tttcctgaaa ggtatcttgg agataggaac agactctcag agcatggtca 60
gaaagccaca gctcatcaat gaaatggtca gggacttcct gtcctgctcc atgcataaat 120
gaaagacgaa gacaactcaa attggcattt gaggggcaga taaacaggag catccggtag 180
tttcacaggt ggtcgggtag caggagccgg gttggttggt tggtctgtgg agagtgcagg 240
gattaaggga agaggcctgg accccaactt cttccttggc tacccccctg aaaatgtcac 300
ctgccttgca tggacgaact cacaggcagg aatgggttgg cttgggtggg gacatcctgc 360
aggttccacc ctcatgttgg ttcatcttca acattgtact gacttcttcc cacttgacat 420
tcctcaaggt cctgtgatca tggctgggtc tagtgaggtt caaacctgca ctgccctacc 480
cacacccaca cccagctcag cgtcagtcag gatcaacaat tacctagaga tcatctttct 540
ggggcttaag cattggtggg agcagatggg atatgagctg gggatttggg aatgggggaa 600
gatatctgct ccccctcccc ctacacccta gccttttaaa aggccttctc aggtcagaga 660
ccaggagaaa agtataggag agatacacaa tggaccagga agaagaaaag ggagagggag 720
gctcagacct tctagacaag gtaagagggc tctggctgac tccaccatcc gcttcttgag 780

gtctcggcac ctgtaattga caagattaat tcatttatag ggcatctaat tagcaagcaa 840
gtctctggag tcccctgacc cagttactat aacacacagg gggtataggt aggagagtat 900
aagagcccct cctcagggca aatgaatgga ttcttagtac tgtcccccaa gagatagtag 960
gtactaggat ttaggggcac ttctgagccc catttccctg gtaagtgtcc caacccccca 1020
aatcaaccca agcctggtct caatctagga cagtggtaga atgctgtccc tagagtcagt 1080
accatgtgaa attgtgctgc aggcaggggc cccaggctgg gaggtggggg ttgggggagt 1140
cagggcaggt cagggaagga gactcaggtt tcatttagag aaattctgca gacccgtgag 1200
gactatggtg agagcagaga tgggaaggca ggcactgttt cgggtggatg ctgtctggaa 1260
gacagggaag gcacagacca aactaaacca atcacgtctg tccccaaggc aggttcaccg 1320
gaccaggaag gcttcttcaa cctgctgacc cacgtgcagg gcgatcgg atg gag gag 1377
Met Glu Glu
1
cag cgc tgt tcc ttg cag gct ggg cca ggc cag aac cca gaa agc c 1423
Gln Arg Cys Ser Leu Gln Ala Gly Pro Gly Gln Asn Pro Glu Ser
    5                   10                  15
gtaagcaggg cgtgattggg ccgtgtgtgt gtgtgtgtgt gtgtgtgtgt gtgtgtgtgt 1483
gtgtggcagg agtgctgggg ttctgggatc ttgtggatct tgggactcag gatggggtct 1543
gtattcatgc ctgcctgtct ctgctccaag cag ag ggt ggc cct gct cca gag 1596
Gln Gly Gly Pro Ala Pro Glu
1 5
atg gac aat ctc atg gat atg ctg gtc aac acc cag ggc cgc cgc atg 1644
Met Asp Asn Leu Met Asp Met Leu Val Asn Thr Gln Gly Arg Arg Met
10 15 20
gac gac cag cgt gta aca gtt aat tcc ctg cct ggc ttc caa cct atc 1692
Asp Asp Gln Arg Val Thr Val Asn Ser Leu Pro Gly Phe Gln Pro Ile
25 30 35
ggc ccc aag gtaggtgatg tccagattac ctgtgagact ccacatagct 1741
Gly Pro Lys
40

ctctaaatct atgacctgtc tctaggcagg aaaggagagg accctatgaa cacgtaaagt 1801
gctatgggct taaggtcagg tggcaggact catgctagtg cagaactatg gctggaaatt 1861
acagttcctg ctccaacatc tgtatatttg ggagaggcca cagggagaaa acaggcagtt 1921
ttcctggaag gcatatgaat gcatacccct ataaatcaat gaagagtagg gcttctgttt 1981
gggagtgttt tgctttattg tttttgagac agggtttcat gtagctctgg ctggcatgtt 2041
ctcctacatg tgcatcctgg gttctgggat aacaggtgtg agtcaccatg agtgatgtat 2101
gtgggtaggg atagaaccca gggctttgat gcagtctcta tcaactgagc tccagcccca 2161
gccctatgtc tgtgtacatt agcatacatg tttagagctc cgggcacacg tgtgcacacg 2221
caggtggagg ccagaagtca atctcctgcc ctgggagctt tcagtgccct ggaactccag 2281
gtagatcagg ctctctagct aggaagccct tgggatcctc ctgactctta agcactgaga 2341
ttacaagtgc ataaacccac acctggctta aactcaggtc ttcaaatgag catagcaagg 2401
atttcaatga ctgagctatc ttctcaactc aactgtttgt ttgtttgttt tagtatttag 2461
ctttgaactc aaaataatcc tcctgcctgt ttcttgagta ctgggattac aggtatacac 2521
taacaggcca atgtctgacc aaataccacc accctaatta gcagacgaaa aaaaaacatt 2581
gtttggaggc acttctgact tgcactttcc ttggtcccct ccctccgtct gacccttctt 2641
catccccag gat gga atg cag aaa cga cct ggg acc ctc agc cct caa 2689
Asp Gly Met Gln Lys Arg Pro Gly Thr Leu Ser Pro Gln
1 5 10
ccc ctg ctc acc cct cag gat cct gct gca ctc agc ttc cgc agg aac 2737
Pro Leu Leu Thr Pro Gln Asp Pro Ala Ala Leu Ser Phe Arg Arg Asn
15 20 25
agc agc ccc cag ccc cag aca caa gct cct tgagagttct agccatcctg 2787
Ser Ser Pro Gln Pro Gln Thr Gln Ala Pro
30 35
ggcctcccac tggcccctga aaacaataaa acacttggca ctagcaacaa agagttgagt 2847
gtgtgttatt ttctgtggtg gggaagggag ctgggacttg aggaactgaa ggtctcagga 2907
gctctgctgg gcagcttgaa gaagtctctc ttctttctgc ttccggatct tctgcttaaa 2967
ttcttctagc tcctggcgct ggaatgggga aaggggtgtg atgggaagga aggaagagta 3027
caggcctcac agcctggact cactcacact atcctccctt tggcttcaga gttcagtatc 3087

cacactggga gccccatgcc aatcacaatc actgtacaag tgagttcagc ttcatccctc 3147
ggggaaaagg taatatgtga caccatttgt gccctcccct ctttttaaga tggggtctca 3207
tatactacag gctagccttg agctcaccag gcggcagaga atagccagaa ttctcaatcc 3267
tcttgcatcc atctcctgag tgctggaatg ctggaattac agcttcctct cctgtctccc 3327
tctctctatc cccatgcagc ccaggctagc ttcaatctga tactcctcct actcctcctt 3387
ccaagtgtcc gtaggtatac accatcacaa acaacaagaa acctttatgg agacaaggtc 3447
tctagcccag gctagtctgg aattcctact cagtctgctg cttccacttt cctacctatg 3507
gctgagggtg aaatctttat tccaagccca actaggtaag agtgactcag ctccttgggg 3567
aaaacaggtt actgacctga ccctccttct ctcttggcca cagctccctc tgtggaacaa 3627
agtcacaggt gagaacacaa ggcaggagaa tccagagccc cacatccaca acagggttga 3687
ctcatgagag gcagacaatg gatctcaata gcaagttggt gcttcatacc ctcccttccg 3747
caggaattat ccatcaagca ctttgatacc caccttacgc tggacaacat agtcctcaaa 3807
ccactcagcc tgattggaga tccagaacat aaccacgggg aaagtgaggt agagggacat 3867
ctgtaaaagc agaggtgggt ggagcacagg gagattgcag ggaagcccaa aggacaggtc 3927
cggagctc 3935

<210> 3
<211> 3279
<212> DNA
<213> Mus musculus
atctctgtct ccaccactca gaggcactca cagactccag ttctgccatc tgtccacata 60
cactgcctgg gttccacctc ccactgacat tcccttgtag gtccccagct tcttccctgg 120
cctcacgtct cccatgggag gtggaggatc agtttaggcg gaatggctgg taggattttg 180
gtggacgtga gagccaatcc tgtggctatg tggttggatc gatcaaacca cggcctctgg 240
gagccgagcc agccgtctgt ctggcagatg atttgggatt tgagagctgc aggttcagat 300
gggaggtgac agtgggctgg gtcctgatgg tgataaagga gagggagaca ccagggcacc 360
tgacaggacc tgacaggggc tatgacagag tggggtgggg ggtgcggagg aggaggcaac 420
catggaaagt tggcttggct gactacagaa aactgaaatg tgtgccaccg gtgctacccc 480
gccctgccac ctctttcctg gacagtcttc ggttacctcc atgtgtctat aacctcacct 540

atctcccaac agcgctgtgg agtattccat tcttcacaaa caagcaaagc tccagcttgc 600

cactaccact gtagtcaagg tggttgccac agcagttgat atcagtgctc tggtccccag 660

ggagcccatc accctccagc ctgcctacag cacagcttta ccagttagga ggcagttgga 720

cacacacact cctgtgtccc ctgttctgag aactgggtgg ggccagaaag gctggaaagg 780

gaggcgggcc ttcaggtggc ctcttctctt ggcatcggag gatccagccc acttgattcc 840

ctgacgctgg tggtagtggt ggcagtggca atcgctgtag cacttgggcc atg gca 896

Met Ala

1

gag gat ggg ccg cag aag cag cag ctg gag atg ccg ctg gtt ctg gac 944

Glu Asp Gly Pro Gln Lys Gln Gln Leu Glu Met Pro Leu Val Leu Asp

        5                   10                  15

cag gac ctg acc cag cag atg cgg ctc cga gta gag agc ctg aag cag 992

Gln Asp Leu Thr Gln Gln Met Arg Leu Arg Val Glu Ser Leu Lys Gln

    20                  25                  30

cgt ggg gag aag aag cag gat ggt gag aag ctg atc cgg ccg gct gag 1040

Arg Gly Glu Lys Lys Gln Asp Gly Glu Lys Leu Ile Arg Pro Ala Glu

35                  40                  45                  50

tcc gtc tac cgc ctc gat ttc atc cag cag cag aag ctg cag ttc gat 1088

Ser Val Tyr Arg Leu Asp Phe Ile Gln Gln Gln Lys Leu Gln Phe Asp

                55                  60                  65

cac tgg aac gtg gtt ctg gac aag ccc ggc aag gtc acc atc acg ggc 1136

His Trp Asn Val Val Leu Asp Lys Pro Gly Lys Val Thr Ile Thr Gly

            70                  75                  80

acc tcg cag aac tgg acg ccc gac ctc acc aac ctc atg aca cgc cag 1184

Thr Ser Gln Asn Trp Thr Pro Asp Leu Thr Asn Leu Met Thr Arg Gln

        85                  90                  95

ctg ctg gac ccc gcc gcc atc ttc tgg cgc aag gaa gac tcc gac gcc 1232

Leu Leu Asp Pro Ala Ala Ile Phe Trp Arg Lys Glu Asp Ser Asp Ala

    100                 105                 110

atg gat tgg aat gag gca gac gcc ctg gag ttt ggg gag cgc ctt tct 1280
Met Asp Trp Asn Glu Ala Asp Ala Leu Glu Phe Gly Glu Arg Leu Ser
115                 120                 125                 130
gat ctg gcc aag atc cgc aag gtc atg tat ttc ctc atc acc ttt ggc 1328
Asp Leu Ala Lys Ile Arg Lys Val Met Tyr Phe Leu Ile Thr Phe Gly
                135                 140                 145
gag ggc gtg gag cct gcc aac cta aag gcc tct gtg gtg ttt aac cag 1376
Glu Gly Val Glu Pro Ala Asn Leu Lys Ala Ser Val Val Phe Asn Gln
            150                 155                 160
ctc tgatgacagc cctggctgcc ctacccctgg ccccacctct cccttgcctg 1429
Leu
gatctccttc ctcatgtgta tttgggggac attcttctag ctgctcctcc tgtgctcatc 1489
ttggccagag ttcccccgag tgctacatcc cctccttttc cctggtgcca gtgctgcggc 1549
tcacagtgat gtcccatggc tccgtagtct agatctagaa gccggatgct gctactatag 1609
actgtagagg ccttttgggt ccacgtggga agatggatgg gccccctgtg gtgaagagcg 1669
ggactgagag ataaagagac tgaccaagag atgcaaacgg ccagcactga ttcctccctt 1729
cagggacggg agactgagac tggacaggaa caccttccgg ggaacctggc aagaaggcgt 1789
ttgccctgct ggccaaagct ggagccagga ggcgaatgcc cagcctctgg cagcaggaag 1849
gttctcctcc cagtgtcggc agcagcccgc tgtgacctta gggccttcaa gacactgggc 1909
aggatgacag cggggcttga tctgactgct tttccaggtc tgggcctggt ttttatggag 1969
aagtgagaga gtgtgtagaa actgaaacaa ctctagccac ccacgctcat atgggtattg 2029
agagatggca taactatttg tatggatgtg ggcctgaggg ctagtcttgg tgaggagtaa 2089
ggctaacttt agtttaatta ttgagctggt actggcttgt gggcttggtg gaggtgatcc 2149
tgactgaggc gtccttggtg cagtgctttt tgaactggga gactgagact cgaatggtgt 2209
agcagagtta gaggggtcca gggctctgag ctagcaacag tgatgtccct gttaggaagg 2269
ctggcatttg ctgctcgctg gtgttgtgcc ctgctgtcac ccccctgggc atatcctggc 2329
tgttctcctg gagtgcagac ccctaagtaa ggcttgggtg ggggcagtta ggatgcctga 2389
cgtctgaagt gggctggagc tatctgactg tgatgcctaa actgacagga aaacggtggc 2449
acagttagca ggttcagctc taccccaagt ctcattgtcc ctcgccttgc acatcctgaa 2509

agccttccat tgcctgttac ctagcatcag ccagaggtac ctcagcagtg tcccctgact 2569
gtctcaaggc tgcctccctc gggcatactg aaggtaggat ctgtcccagc tggtgagctg 2629
ccaggactgc aaaccccagc tcaggtgcag gattctggag gcaggagata ggctgtggta 2689
ccggtgtctc ttgagccggt gcctctgctc cataacatgc ttgccgaagc actggccggt 2749
gcttctggat tctgctgact ctagggagcc acacccagac agtgcctctg cctttctgct 2809
tctcttcctg acctctccct acagctttag agaccccttt ggttcacact gcctgtgccc 2869
caactctgcc tcactcggat ccgtctgccc tgtggggaca tgagtgtctc tgttgtgcct 2929
gtttcacaat aaagactgtg tgccctcccc tctgtggtgt ggtgtgtgtg cctccgtggt 2989
gtggtttgca catcttgctg caagcccata gcatcagaat ccttctctca tgggccctgt 3049
agctctgagc aactccaccc tgccagcctt gaggatgagg ccgagtcgtg agatctctca 3109
tgaggattga gtttcacctg tcagccaggt ttcctggctg ccctgcaggt accaatcctc 3169
tagggtatga aagagcatgc taaagctatg cttggggcag gggagtgtag cgggtaggac 3229
tgatactaat ttagcttggt cttggtcact gtttggctgt gccctctaga 3279

## Claims

1. A transgenic animal into which a gene encoding a trans-synaptic tracer protein is introduced so as to direct specific expression in particular neurons.

2. The transgenic animal according to claim 1, wherein a promoter specific to the particular neurons is located upstream of the gene encoding the trans-synaptic tracer protein.

3. The transgenic animal according to claim 1 or 2, wherein the trans-synaptic tracer protein is wheat germ agglutinin.

4. A method for screening neuromimetic substances, which comprises:
administering a test substance to the transgenic animal according to any one of claims 1 to 3; and
selecting a neuromimetic substance from among the test substances by using as an indicator the trans-synaptic tracer protein expressed in neurons of the transgenic animal.

5. A neuromimetic substance obtainable by the screening method according to claim 4.
